(19) Europäisches Patentamt
European Patent Office
Office européen des brevets

(11) Veröffentlichungsnummer : **0 108 033**
**B1**

(12) # EUROPÄISCHE PATENTSCHRIFT

(45) Veröffentlichungstag der Patentschrift : **02.12.87**

(51) Int. Cl.[4] : **C 07 J 53/00**, A 61 K 31/585

(21) Anmeldenummer : **83730098.7**

(22) Anmeldetag : **14.10.83**

(54) **Neue 7-alpha-Acylthio-1-alpha,2-alpha-Methylen-3-oxo-17-alpha-pregn-4-en-21,17-carbolactone, deren Herstellung und diese enthaltende Arzneimittel.**

(30) Priorität : **29.10.82 DE 3240510**

(43) Veröffentlichungstag der Anmeldung : **09.05.84 Patentblatt 84/19**

(45) Bekanntmachung des Hinweises auf die Patenterteilung : **02.12.87 Patentblatt 87/49**

(84) Benannte Vertragsstaaten : **AT BE CH DE FR GB IT LI LU NL SE**

(56) Entgegenhaltungen :
**DE-A- 2 652 761**
**US-A- 3 013 012**
**US-A- 3 753 979**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Laurent, Henry, Dr.**
**Glambecker Weg 21**
**D-1000 Berlin 28 (DE)**
Erfinder : **Nickisch, Klaus, Dr.**
**Alt-Lichtenrade 11**
**D-1000 Berlin 49 (DE)**
Erfinder : **Bittler, Dieter**
**Bölkauer Pfad 11**
**D-1000 Berlin 27 (DE)**
Erfinder : **Wiechert, Rudolf, Prof. Dr.**
**Petzower Strasse 8A**
**D-1000 Berlin 39 (DE)**
Erfinder : **Losert, Wolfgang, Prof. Dr.**
**Landshuter Strasse 22**
**D-1000 Berlin 30 (DE)**

**EP 0 108 033 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 7α-Acylthio-1α,2α-Methylen-3-oxo-17α-pregn-4-en-21,17-carbolactone (I) sowie Verfahren zu ihrer Herstellung und diese enthaltende pharmazeutische Präparate gemäß der Patentansprüche.

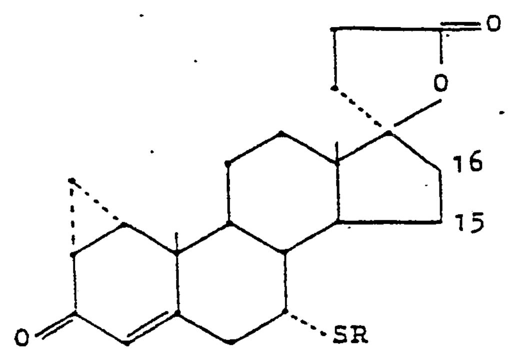

(I)

worin

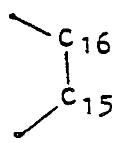
die Gruppierung
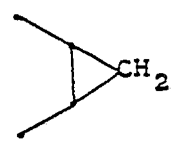
oder
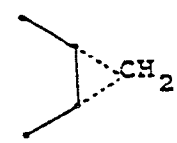

und R einen niederen gesättigten Acylrest mit 2 bis 4 Kohlenstoffatomen bedeuten.

R in der Bedeutung eines niederen gesättigten Acylrestes stellt den Acetyl-, Propionyl- oder Butyrylrest dar, bevorzugt sind der Acetyl- und der Propionylrest.

Die in dem US-Patent 3,735,979 beschriebenen 1α,2α-Methylen-6α,7α-dihalogenmethylen-androst-4-en-3-one besitzen starke antiandrogene Eigenschaften. Wie man aus J. Med. Chem. 15, 1165 (1972) jedoch entnehmen kann, sind sie als Aldosteronantagonisten nur schwach wirksam. Die wirksamste Verbindung aus der US-Patentschrift 3 013 012 ist das bekannte Spironolacton, das aber im Vergleich zu den erfindungsgemäß Verbindungen eine geringere Antialdosteronwirkung und einen stärkeren antiandrogenen Effekt zeigt. Die deutsche Offenlegungsschrift 2 652 761 beschreibt in Beispiel 7 ein 7α-Acetylthio-15β,16β-methylen-3-oxo-4-androsten-[17(β-1')-spiro-5'] perhydrofuran-2'-on, das ähnlich wie die erfindungsgemäßen Verbindungen an den Androgenrezeptor bindet, aber eine deutlich schwächere Antialdosteronwirkung besitzt.

Die neuen Verbindungen der allgemeinen Formel I haben die Eigenschaften, die Wirkung von Aldosteron oder Desoxycarticosteron auf die Natrium- und Kaliumsalzausscheidung aufzuheben bzw. umzukehren. Die erfindungsgemäßen Verbindungen sind damit geeignet zur Behandlung von bestimmten Formel der Hypertonie, von Ödemen, des primären Aldosteronismus und anderer durch Aldosteron bedingter endokrinologischer Störungen. Sie können außerdem als Diuretika eingesetzt werden.

Bei der Behandlung mit Spironolacton treten häufig unerwünbschte endokrine Nebenwirkungen auf, die durch die anti-androgene und gestagene Aktivität von Spironolacton hervorgerufen werden. So beobachtete man bei länger andauernder Behandlung von männlichen Patienten mit Spironolacton das Auftreten von Gynäkomastie (Smith, W.G., The Lancet 1962, S. 886 ; Mann, N.M., JAMA 1963, S. 778 ; Clark, E., JAMA 1965, S. 157 ; Greenblatt, D.J., JAMA 1973, S. 82) und Impotenz (Greenblatt, D.J., JAMA 1973, S. 82), was auf die antiandrogene Nebenwirkung dieses Wirkstoffes zurückgeführt wird (Steelman, S.L. et al., Steroids 1963, S. 449 ; Schane, H.P., J. of Clinical Endocrinology and Metabolism 1978, S. 691).

Die bei Frauen unter der Behandlung mit Spironolacton auftretenden Nebenerscheinungen wie Amenorrhoe und Cyclus-Unregelmäßigkeiten werden dagegen der gestagenen Nebenwirkung von Spironolacton angelastet. Beide Nebenwirkungen lassen sich sowohl in Tierversuchen als auch in vitro durch den Rezeptorbindungstest mit dem Androgen- bzw. Gestagen-Rezeptor nachweisen.

Es war Aufgabe der vorliegenden Erfindung, Verbindungen zur Verfügung zu stellen, die dem Spironolacton in der Antialdosteronwirkung überlegen sind und dabei möglichst verminderte, höchstens jedoch vergleichbare endokrine Nebenwirkungen zeigen.

Die Antialdosteronwirkung wurde im Testmodell von Hollmann (G. Hollmann et al., Tubuläre Wirkungen und renale Elimination von Spironolactonen, Naunyn-Schmiedebergs Arch. Exp. Path. Pharmak. 247 (1964), S. 419 ; P. Marx, Renale Wirkungen des d-Aldosterons und seines Antagonisten Spironolacton, Diss. Med. Fak. FU Berlin, 1966) festgestellt und gemessen.

Der Androgen-Rezeptor-Bindungstest wird wie folgt durchgeführt :

Der im Cytosol eines Homogenats von Rattenprostata enthaltene Androgen-Rezeptor (Protein) bindet Dihydrotestosteron (DHT) mit hoher Affinität aber niedriger kapazität. Wird dieser Rezeptor beladen mit $^3$H-DTH in Gegenwart der zu prüfenden Verbindung, so hängt es von der Konzentration und von der Bindungsaffinität der zu prüfenden Verbindung ab, wie stark $^3$H-DHT vom Rezeptor verdrängt wird. Nach Trennung des Rezeptor-gebundenen DHT vom nichtgebundenen, kann man die Bindung in Prozent ermitteln und trägt diesen Wert auf gegen den Logarithmus der molaren Konzentration der Prüfsubstanz. Man ermittelt nun die Konzentration der Prüfsubstanz, die erforderlich ist, um die Referenz-Substanz vollständig vom Rezeptor zu verdrängen. Der Kompetitionsfaktor (KF) als Maß für die Bindungsstärke ist definiert als das Verhältnis der Konzentration der Prüfsubstanz zur Konzentration der Referenzsubstanz, so daß ein hoher KF-Wert geringe Bindungsstärke, ein niedriger aber hohe Affinität anzeigt.

Die antiandrogene Wirkung wird bei Verbindungen festgestellt, die selber zwar keine androgene Wirkung besitzen, aber durch ihre hohe Bindungsaffinität das körpereigene Androgen vom Rezeptor ganz oder teilweise verdrängen, wie das im gewissen Maß bei Spironolacton beobachtet wird. Erwünscht ist deshalb ein hoher Kompetitionsfaktor im Androgen- und Gestagen-Rezeptor-Test.

Der Gestagen-Rezeptor-Bindungstest erfolgt in gleicher Weise unter Verwendung von Cytosol aus Rattenuterus-Homogenat.

In der nachfolgenden Tabelle sind zusammengestellt die relativen Werte der Antialdosteron-Wirkungsstärke (mit Spironolacton = 1) und die Kompetitionsfaktoren im Androgenrezeptortest ($K_A$) und im Gestagenrezeptortest ($K_G$) von Spironolacton und den erfindungsgemäßen Verbindungen am Beispiel von

7α-Acetylthio-1α,2α ; 15β,16β-dimethylen-3-oxo-17α-pregn-4-en-21,17-carbolacton (B)

und

7α-Acetylthio-1α,2α ; 15α,16α-dimethylen-3-oxo-17α-pregn-4-en-21,17-carbolacton (C)

Tabelle

| Verbindung | relative Antialdosteron-Wirkung | Kompetitionsfaktor | |
|---|---|---|---|
| | | $K_A$ | $K_G$ |
| Spironolacton | 1 | 8,9 | 21 |
| B | 2 - 3 | 15 | 7,8 |
| C | 3 - 4 | 16 | 19 |

Die Tabelle zeigt, daß die erfindungsgemäßen Verbindungen eine erheblich stärkere Antialdosteronaktivität besitzen, ohne daß die endokrinen Nebenwirkungen ebenfalls stärker geworden sind ; sie liegen in der Größenordnung von Spironolacton bzw. sogar darunter.

Die Herstellung der erfindungsgemäßen Verbindungen der Formel I erfolgt gemäß Anspruch 4 nach an sich bekannten Methoden. Besonders geeignet ist die Umsetzung von Verbindungen der allgemeinen Formel II mit der entsprechenden Thiosäure Acyl-SH in Gegenwart eines protischen Lösungsmittels wie Methanol oder Äthanol, auch im Gemisch mit Wasser bei einer Temperatur von Raumtemperatur bis zum Siedepunkt des Reaktionsgemisches. Man erhält so nach geeigneter Aufarbeitung die erfindungsgemäßen Verbindungen der Formel I mit R in der Bedeutung eines Acylrestes. Es ist jedoch auch möglich, die Addition der Thiocarbonsäure ohne Lösungsmittel durchzuführen.

Die zur Durchführung der erfindungsgemäßen Reaktion benötigten Ausgangsverbindungen der Formel II werden wie folgt hergestellt :

1. 1α,2α ; 15β,16β-Dimethylen-3-oxo-17α-pregna-4,6-dien-21,17-carbolacton

12,0 g 15β,16β-Methylen-3-oxo-17α-pregna-4,6-dien-21,17-carbolacton werden in 500 ml Benzol gelöst und mit 12,0 g Dichlordicyanochinon 20 Stunden am Rückfluß gekocht. Nach dem Abkühlen wird das ausgefallene Hydrochinon abfiltriert, das Filtrat mit Ether verdünnt, mit Natriumhydrogencarbonatlösung und Wasser gewaschen, über Magnesiumsulfat getrocknet und im Vakuum eingeengt. Der Rückstand wird durch Säulenchromatographie an Kieselgel gereinigt. Man erhält 9,5 g 15β,16β-Methylen-3-oxo-17α-pregna-1,4,6-trien-21,17-carbolacton.

UV : $\varepsilon_{222}$ = 11 500 ; $\varepsilon_{255}$ = 9 700 ; $\varepsilon_{299}$ = 11 800.

Zu einer Lösung von 14,36 g Trimethylsulfoxoniumiodid in 120 ml Dimethylsulfoxid gibt man 2,63 g 55 %iges Natriumhydrid und rührt 1 Stunde bei Raumtemperatur. Dazu tropft man 9,3 g 15β,16β-Methylen-3-oxo-17α-pregna-1,4,6-trien-21,17-carbolacton in 40 ml DMSO, rührt 1 Stunde bei Raumtemperatur nach

und fällt anschließend in Eiswasser. Der erhaltene Niederschlag wird abfiltriert, mit Wasser nachgewaschen, in $CH_2Cl_2$ aufgenommen, mit Magnesiumsulfat getrocknet und im Vakuum eingeengt. Das erhaltene Rohprodukt wird durch Säulenchromatographie an Kieselgel gereinigt. Man erhält 5,5 g 1α,2α ; 15β,16β-Dimethylen-3-oxo-17α-pregna-4,6-dien-21,17-carbolacton.

UV : $\varepsilon_{282} = 20\,500$

Fp. : 203-205 °C

2. 1α,2α ; 15β,16β-Dimethylen-3-oxo-17α-pregna-4,6-dien-21,17-carbolacton

5,0 g 15α,16α-Methylen-3-oxo-17α-pregna-4,6-dien-21,17-carbolacton werden 50 ml Dioxan mit 5,0 g 2,3-Dichlor-5,6-dicyan-p-benzochinon 3 Stunden bei 100 °C gerührt. Die Reaktionslösung wird dann abekühlt, das ausgefallene Hydrochinon abgesaugt und mit Dioxan nachgewaschen. Das Filtrat wird im Vakuum weitgehend eingeengt. Der Rückstand wird in Ether aufgenommen, mit Natriumhydrogencarbonatlösung und Wasser gewaschen, getrocknet und eingedampft. Nach Chromatographie an Kieselgel werden 3,46 g 15α,16α-Methylen-3-oxo-17α-pregna-1,4,6-trien-21,17-carbonlacton erhalten.

UV : $\varepsilon_{222} = 11\,250$ ; $\varepsilon_{254} = 9\,140$ ; $\varepsilon_{299} = 11\,500$.

8,8 g Trimethylsulfoxoniumiodid werden in 99 ml Dimethylsulfoxid mit 1,391 g Natriumhydrid, eine 55 %ige Ölsuspension, bis zur Lösung des Hybrids gerührt. Dann werden unter Argon 2,8 g 15α,16α-Methylen-3-oxo-17α-pregna-1,4,6-trien-21,17-carbolacton zugegeben und 2 Stunden bei Raumtemperatur nachgerührt. Die Reaktionslösung wird in Eiswasser eingerührt, mit 2n Schwefelsäure schwach angesäuert und der ausgefallene Niederschlag abfiltriert. Nach dem Auflösen in Methylenchlorid wird mit Wasser gewaschen, getrocknet und eingedampft. Der Rückstand wird an Kieselgel chromatographiert, und es werden 2,1 g 1α-2α ; 15α,16α-Dimethylen-3-oxo-17α-pregna-4,6-dien-21,17-carbolacton erhalten.

UV : $\varepsilon_{281} = 19\,500$.

Die erfindungsgemäßen Verbindungen können nach an sich bekannten Methoden der Galenik zu Arzneimitteln für die orale oder parenterale Applikation formuliert werden. Sie können in der gleichen Weise dosiert und appliziert werden wie das handelsübliche Spironolacton.

Für die Anwendung am Menschen beträgt die Dosierung 20 bis 500 mg pro Tag.

Beispiel 1

Zu einer Lösung von 4,7 g 1α,2α ; 15β,16β-Dimethylen-3-oxo-17α-pregna-4,6-dien-21,17-carbolacton in 15 ml Methanol tropft man bei 60 °C 2 ml Thioessigsäure und rührt für 5 Stunden bei dieser Temperatur. Nach dem Abkühlen verdünnt man mit Chloroform, wäscht mit Natriumhydrogencarbonat und Wasser, trocknet über Magnesiumsulfat und engt im Vakuum ein. Das erhaltene Rohprodukt wird durch Säulenchromatographie an Kieselgel gereinigt. Man erhält 2,84 g 7α-Acetylthio-1α,2α ; 15β,16β-dimethylen-3-oxo-17α-pregna-4-en-21,17-carbolacton.

UV : $\varepsilon_{235} = 16\,600$.

Fp. : 257-259 °C.

Beispiel 2

1,0 g 1α,2α ; 15β,16β-Dimethylen-3-oxo-17α-pregna-4,6-dien-21,17-carbolacton werden in 20 ml Methanol mit 4 ml Wasser und 1,5 mol Thioessigsäure 16 Stunden bei Raumtemperatur gerührt. Die Reaktionslösung wird dann mit Ether verdünnt, mit Natriumhydrogencarbonatlösung und Wasser gewaschen, getrocknet und eingedampft. Der Rückstand wird an Kieselgel chromatographiert, und es werden, nach Diisopropylether-Verreibung, 460 mg 7α-Acetylthio-1α,2α ; 15α,16α-dimethylen-3-oxo-17α-pregn-4-en-21,17-carbolacton vom Schmelzpunkt 246 °C Zers. erhalten.

UV : $\varepsilon_{234} = 15\,600$.

**Patentansprüche**

1. 7α-Acylthio-1α,2α-Methylen-3-oxo-17α-pregn-4-en-21,17-carbolactone der allgemeinen Formel I

(Siehe Formel I Seite 5 f.)

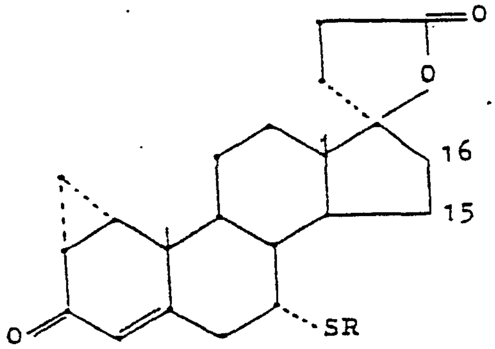

(I)

worin

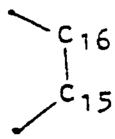 die Gruppierung 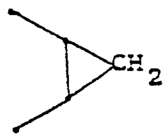 oder 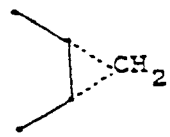

und R einen niederen gesättigten Acylrest mit 2 bis 4 Kohlenstoffatomen bedeuten.

2. 7α-Acetylthio-1α,2α ; 15β,16β-di-methylen-3-oxo-17α-pregn-4-en-21,17-carbolacton.

3. 7α-Acetylthio-1α,2α ; 15α,16α-di-methylen-3-oxo-17α-pregn-4-en-21,17-carbolacton.

4. Verfahren zur Herstellung von 7α-Acylthio-1α,2α-Methylen-3-oxo-17α-pregn-4-en-21,17-carbolactonen der allgemeinen Formel I, dadurch gekennzeichnet, daß man in an sich bekannter Weise an eine Verbindung der allgemeinen Formel II

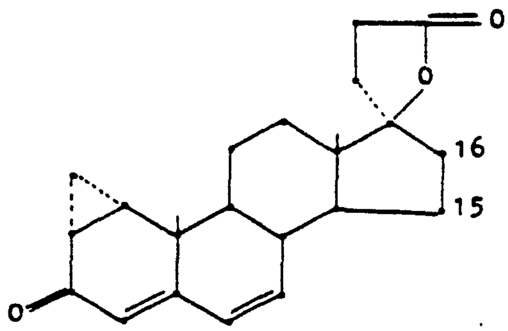

(II)

worin

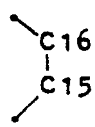 die Gruppierung 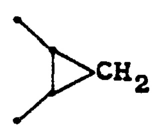

oder

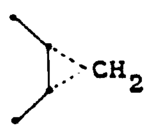

bedeutet, eine Thiocarbonsäure der allgemeinen Formel III

R'—S—H (III)

worin R' einen niederen, gesättigten Acylrest mit 2 bis 4 Kohlenstoffatomen bedeutet, bei einer Temperatur von Raumtemperatur bos Siedepunkt in einem polaren, protischen Lösungsmittel addiert.

5. Arzneimittel mit Antialdosteron-Wirkung, das eine Verbindung gemäß der Ansprüche 1 bis 3 enthält.

**Claims**

1. 7α-Acylthio-1α,2α-methylen-3-oxo-17α-pregn-4-ene-21,17-carbolactone of the general formula I

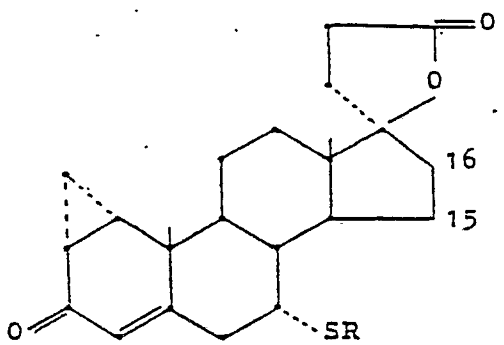

(I)

wherein

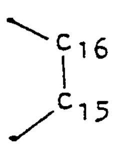 is the group 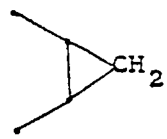 or 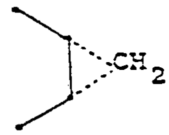

and R is a lower saturated acyl group of 2 to 4 carbon atoms.

2. 7α-Acetylthio-1α,2α ; 15β,16β-dimethylen-3-oxo-17α-pregn-4-ene-21,17-carbolactone.

3. 7α-Acetylthio-1α,2α ; 15α,16α-dimethylen-3-oxo-17α-pregn-4-ene-21,17-carbolactone.

4. A process for the preparation of 7α-acylthio-1α,2α-methylen-3-oxo-17α-pregn-4-ene-21,17-carbolactones of the general formula I characterised in that there is added, in known manner, to a compound of general formula II.

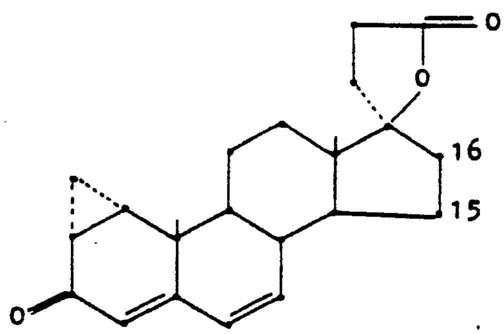

(II)

wherein

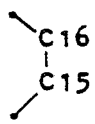 is the group 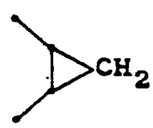

or

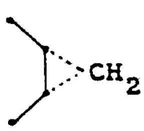

a thiocarboxylic acid of general formula III

$$R'—S—H \qquad (III)$$

wherein R is a lower saturated acyl group of 2 to 4 carbon atoms, at a temperature of from room temperature to boiling point in a polar, protic solvent.

5. Pharmaceutical composition with antialdosterone activity which contains a compound according to claims 1 to 3.

**Revendications**

1. Acylthio-7α méthylène-1α,2α oxo-3 17α-prégnène-4 carbolactones-21,17 qui répondent à la formule générale I

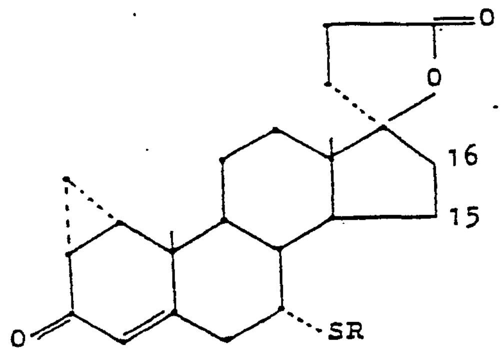

(I)

dans laquelle

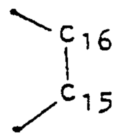 représente un groupement 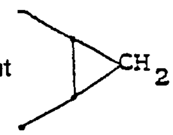 ou 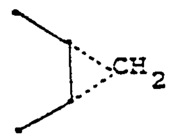

et R représente un radical acyle inférieur saturé qui contient de 2 à 4 atomes de carbone.

2. Acétylthio-7α diméthylène-1α,2α ; 15β,16β oxo-3 17α-prégnène-4 carbolactone-21,17.

3. Acétylthio-7α diméthylène-1α,2α ; 15α,16α oxo-3,17α-prégnène-4 carbolactone-21,17.

4. Procédé de préparation d'acylthio-7α méthylène-1α,2α oxo-3 17α-prégnène-4 carbolactones-21,17 qui répondent à la formule générale I représentée et définie à la revendication 1, procédé caractérisé en ce qu'on fixe, de manière connue, sur un composé répondant à la formule générale II :

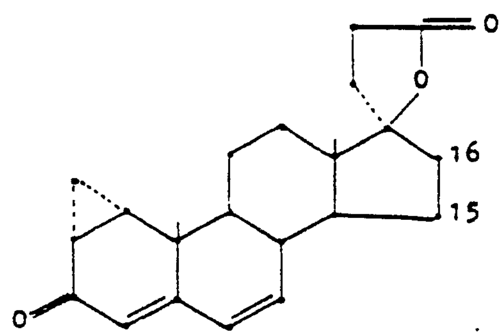

(II)

dans laquelle

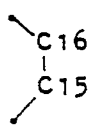 représente un groupement 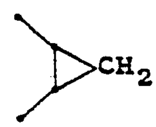

ou

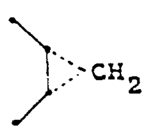

un acide thiocarboxylique répondant à la formule générale III :

R'—S—H (III)

dans laquelle R' représente un radical acyle inférieur saturé qui contient de 2 à 4 atomes de carbone, à une température comprise entre la température ambiante et le point d'ébullition, dans un solvant protique polaire.

5. Médicament à action anti-aldostérone qui contient un composé selon l'une quelconque des revendications 1 à 3.